# EUROPÄISCHE PATENTSCHRIFT

(11) **EP 0 686 634 B1**
(45) Veröffentlichungstag und Bekanntmachung des Hinweises auf die Patenterteilung: **19.07.2000**
(21) Anmeldenummer: 95107118.2
(22) Anmeldetag: 11.05.1995
(51) Int. Cl.: C07D 309/12

(54) **Verfahren zur Herstellung von cyclischen Etherketonen**
Process for the production of cyclic etherketones
Procédé pour la préparation d'éthercétones cycliques

(30) Priorität: 20.05.1994 DE 4417696
(43) Veröffentlichungstag der Anmeldung: 13.12.1995
(73) Patentinhaber: BASF AKTIENGESELLSCHAFT, 67056 Ludwigshafen (DE)
(72) Erfinder: Schnurr, Werner, Dr., D-67273 Herxheim (DE); Fischer, Rolf, Dr., D-69121 Heidelberg (DE)

(56) Entgegenhaltungen:
- EP-A- 0 011 098
- GB-A- 1 133 882
- US-A- 3 496 197
- US-A- 4 120 824
- K. HEYNS, H. BUCHHOLZ: "Über selektive katalytische Oxidationen, XXXVII. Platin-Katalyse als Hydridmechanismus: Die katalytische Oxidation stickstoff- und sauerstoffhaltiger Heterocyclen", CHEM. BER., , 1976, Band 109, Nr. , Seiten 3707 - 3727
- P. LI, H. ALPER: "Cobalt-catalyzed oxidation of ethers using oxygen", J. MOL. CAT., , 1992, Band 72, Nr. , Seiten 143 - 152
- A. SEN ET AL.: "C-H Activation in aqueous Medium. The Diverse Roles of Platinum(II) and Metallic Platinum in the Catalytic and Stoichiometric Oxidative Functionalization of Organic Substrates Including Alkanes", J. AM. CHEM. SOC., , 1992, Band 114, Nr. 16, Seiten 6385 - 6392
- KIRK-OTHMER: "Encyclopedia of Chemical Technology, Bd. 9, S. 864-865", , WILEY-INTERSCIENCE, NEW YORK
- J. FALBE, M. REGITZ: "Römpp Chemie Lexikon, S. 1247-1248", 1990, GEORG THIEME VERLAG, STUTTGART
- P. MüLLER: "The Chemistry of Functional Groups (S. Patai, Hrsg.): Supplement E: The chemistry of ethers, crown ethers, hydroxyl groups and their sulphur analogs: Chapter 12: Oxidation and reduction of alcohols and ethers, S. 469", 1980, JOHN WILEY & SONS, CHICHESTER

## Beschreibung

Die vorliegende Erfindung betrifft ein Verfahren zur Herstellung von cyclischen Etherketonen aus cyclischen Etheraldehyden mit Sauerstoff enthaltenden Gasen in Gegenwart eines Voll- oder Trägerkatalysators, der Kupfer und/oder Mangan enthält, bei erhöhten Temperaturen.

Aldehyde wie Isobutyraldehyd oder Aldehydsteroide lassen sich durch oxidative Decarbonylierung in der Flüssigphase in Ketone überführen. Als Katalysatoren werden z.B. Kupfer(II)acetat in Gegenwart einer stickstoffhaltigen Base (DE-A-17 68 116, GB-A-1 133 882) oder Molybdänoxid (DE-A-19 56 018) eingesetzt.

Die Umsetzung von Isobutyraldehyd zu Aceton in der Gasphase an Kupfer- oder Mangan-Trägerkatalysatoren mit hohen Ausbeuten ist aus DE-A-28 02 672, DE-A-27 38 269, DE-A-24 15 151 und DE-A-21 57 307 bekannt. Die Methode konnte an vergleichbaren Katalysatorsystemen auf andere aliphatische Aldehyde übertragen werden (DE-A-28 48 400).

Er war weiterhin bekannt, daß cyclische Ether schon unter milden Reaktionsbedingungen (ab 25°C) in alpha-Stellung zu Lactonen oxidiert werden (Chem. Ber. 109 (1976) 3707 bis 3727; J. Mol. Cat. 72 (1992) 143 bis 152; J. Am. Chem. Soc. 114 (1992) 6385 bis 6392).

Der vorliegenden Erfindung lag daher die Aufgabe zugrunde, den zuvor genannten Nachteilen abzuhelfen.

Demgemäß wurde ein neues Verfahren zur Herstellung von cyclischen Etherketonen der allgemeinen Formel I 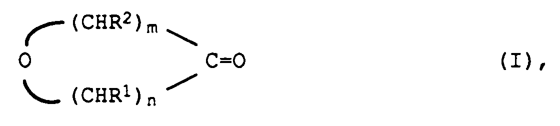 in der R¹ und R² Wasserstoff oder C₁- bis C₄-Alkyl, m und n ganze Zahlen von 1 bis 5 bedeuten, gefunden, welches dadurch gekennzeichnet ist, daß man cyclische Etheraldehyde der allgemeinen Formel II 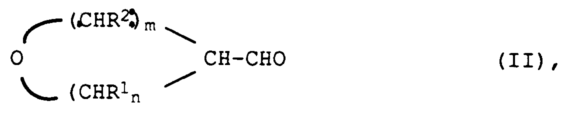 in der R¹, R², m und n die obengenannten Bedeutungen haben, mit Sauerstoff enthaltenden Gasen in Gegenwart eines Voll- oder Trägerkatalysators, der Kupfer und/oder Mangan enthält, bei Temperaturen von 50 bis 300°C und Drücken von 0,01 bis 10 bar umsetzt.

Als Einsatzstoffe dienen formylsubstituierte cyclische Ether mit einer Ringgröße von 4 bis 12 (m und n = 1 bis 5), vorzugsweise von 5 bis 7. Besonders bevorzugt ist 4-Formyltetrahydropyran.

Die Substituenten R¹ und R² und die Indizes m und n haben folgende Bedeutungen:
R¹ und R²
   - Wasserstoff oder
   - C₁- bis C₄-Alkyl wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
m und n
   - eine ganze Zahl von 1 bis 5, bevorzugt 1 bis 4, besonders bevorzugt 1 bis 3.

Als Katalysatoren eignen sich solche, die Kupfer und/oder Mangan enthalten. Es kann sich um Voll- oder Träger-katalysatoren handeln. Die Summe des Kupfer- und/oder Mangan-Gehaltes sollte in der Regel 0,1 bis 50 Gew.-%, bevorzugt 1 bis 20 Gew.-%, besonders bevorzugt 1,5 bis 10 Gew.-% betragen. Positiv auf die Selektivität wirkt sich der Zusatz von Zinkoxid in der Regel in Mengen von 0,4 bis 40 Gew.-%, bevorzugt 1 bis 20 Gew.-%, besonders bevorzugt 2 bis 10 Gew.-% aus.

Als geeignete Trägermaterialien haben sich Aluminiumoxid, Siliziumdioxid, Titandioxid, Zirkoniumdioxid, Aluminiumsilikate, Magnesiumsilikate oder deren Gemische erwiesen. Bevorzugt werden Aluminiumoxid, Titandioxid und Magnesiumsilikat eingesetzt. Derartige Katalysatoren können in bekannter Weise durch Tränken vorgeformter Träger wie Pellets, Kugeln oder Stränge z.B. mit einer wäßrigen Lösung der Metallsalze, die beim Erhitzen in deren Oxide übergehen, hergestellt werden.

Die bevorzugt verwendeten Trägerkatalysatoren zeigen über einen längeren Zeitraum hohe Aktivität. Verbrauchte Katalysatoren lassen sich durch Behandlung mit sauerstoffenthaltenden Gasen z.B. Luft, bei Temperaturen von 350 bis 500°C regenerieren.

Bei der oxidativen Decarbonylierung hält man eine Temperatur von 50 bis 300°C, vorzugsweise 100 bis 250°C, insbesondere von 120 bis 200°C ein. Die Oxidation kann bei Drücken von 0,01 bis 10 bar, bevorzugt 0,1 bis 2 bar, besonders bevorzugt Normaldruck (Atmosphärendruck) durchgeführt werden.

In der Regel arbeitet man bei einer Katalysatorbelastung von 0,01 bis 10, vorzugsweise 0,01 bis 3 kg Aldehyd je kg Katalysator und Stunde.

Als Oxidationsmittel eignen sich Sauerstoff enthaltende Gase, bevorzugt Luft. Die Sauerstoffkonzentration im Eingangsgas richtet sich nach der Aldehydkonzentration. Das molare Verhältnis Sauerstoff im sauerstoffenthaltenden Gas zum Aldehyd beträgt in der Regel 1 : 1 bis 200 : 1, bevorzugt 1,1 : 1 bis 10 : 1, besonders bevorzugt 1,5 : 1 bis 5 : 1.

Vorteilhaft ist auch der Zusatz eines inerten Verdünnungsmittels. In der Regel wird Stickstoff, Wasser, Kohlenmonoxid oder Kohlendioxid verwendet. Bevorzugt wird Stickstoff und/oder Wasserdampf zugegeben.

Die Umsetzung kann in der Flüssigphase, bevorzugt in der Gasphase, diskontinuierlich oder bevorzugt kontinuierlich als Festbettreaktion mit fest angeordnetem Katalysator, beispielsweise in Sumpf- oder Rieselfahrweise oder als Wirbelbettreaktion mit in auf- und abwirbelnder Bewegung befindlichem Katalysator durchgeführt werden.

Mit Hilfe des erfindungsgemäßen Verfahrens ist es möglich, bisher schwer zugängliche Ketone auf einfache Weise selektiv herzustellen.

### Beispiele

### Beispiel 1 (Katalysatorherstellung)

Eine Lösung von 25,1 g Zink(II)nitrat in 70 ml Wasser wird zu 150 g Aluminiumoxid (1,5 mm Stränge) gegeben. Nach Trocknen bei 110°C (15 h) werden die Stränge 15 Stunden bei 350°C mit Luft getempert. Anschließend wird eine Lösung von 24,5 g Kupfer-(II)-nitrat in 70 ml Wasser zu dem vorbehandelten Aluminiumoxid gegeben, getrocknet und kalziniert (s.o.). Der fertige Katalysator enthält 4,1 Gew.-% Kupfer und 3,7 Gew.-% Zink.

### Beispiel 2 (Katalysatorherstellung)

Eine Lösung von 15,2 g Kupfer-(II)-nitrat in 47 ml Wasser wird zu 100 g Aluminiumoxid (1,5 mm Stränge) gegeben. Nach Trocknen bei 110°C (15 h) werden die Stränge 15 Stunden bei 350°C mit Luft getempert. Der fertige Katalysator enthält 3,8 Gew.-% Kupfer.

### Beispiel 3 (Katalysatorherstellung)

Eine Lösung von 18,3 g Mangan-(II)-nitrat in 47 ml Wasser wird zu 100 g Aluminiumoxid (1,5 mm Stränge) gegeben. Nach Trocknen bei 110°C (15 h) werden die Stränge 15 Stunden bei 350°C mit Luft getempert. Der fertige Katalysator enthält 3,7 Gew.-% Mangan.

### Beispiel 4 (Katalysatorherstellung)

Eine Lösung von 18,3 g Mangan-(II)-nitrat in 47 ml Wasser wird zu 100 g Aluminiumoxid (1,5 mm Stränge) gegeben. Nach Trocknen bei 110°C (15 h) werden die Stränge 15 Stunden bei 350°C mit Luft getempert. Anschließend wird eine Lösung von 16 g Zink-(II)-nitrat in 47 ml Wasser zu dem vorbehandelten Aluminiumoxid gegeben, getrocknet und kalziniert (s.o.). Der fertige Katalysator enthält 3,5 Gew.-% Mangan und 3,7 Gew.-% Zink.

### Beispiel 5

Pro Stunde wurden 11 g einer Lösung, die aus 20 Gew.-% 4-Formyltetrahydropyran und 80 Gew.-% Wasser bestand, in einen Verdampfer (200 bis 250°C) gepumpt und von dort mit einem Gemisch aus 17,5 l/h Stickstoff und 2,5 l/h Sauerstoff bei einer Reaktortemperatur von 150°C über 66 g Katalysator (Beispiel 1) in Rieselfahrweise geleitet. Der gasförmige Reaktionsaustrag wurde in Kühlfallen kondensiert und gaschromatographisch analysiert (quantitative Gaschromatographie). Es wurden 85 mol-% Tetrahydropyran-4-on gebildet. Der Aldehyd-Umsatz betrug über 99 %.

### Beispiel 6

Pro Stunde wurden 3 g 4-Formyltetrahydropyran in einen Verdampfer (200 bis 250°C) gepumpt und von dort mit einem Gemisch aus 32,5 l/h Stickstoff und 2,5 l/h Sauerstoff bei einer Reaktortemperatur von 150°C über 66 g Katalysator (Beispiel 1) in Rieselfahrweise geleitet. Der gasförmige Reaktionsaustrag wurde in Kühlfallen kondensiert und gaschromatographisch analysiert (quantitative Gaschromatographie). Es wurden 81 mol-% Tetrahydropyran-4-on gebildet. Der Aldehyd-Umsatz betrug über 87 %.

### Beispiel 7

Pro Stunde wurden 13 g einer Lösung, die aus 20 Gew.-% 4-Formyltetrahydropyran und 80 Gew.-% Wasser bestand, in einen Verdampfer (200 bis 250°C) gepumpt und von dort mit einem Gemisch aus 17,5 l/h Stickstoff und 2,5 l/h Sauerstoff bei einer Reaktortemperatur von 150°C über 68 g Katalysator (Beispiel 2) in Rieselfahrweise geleitet. Der gasförmige Reaktionsaustrag wurde in Kühlfallen kondensiert und gaschromatographisch analysiert (quantitative Gaschromatographie). Es wurden 75 mol-% Tetrahydropyran-4-on gebildet. Der Aldehyd-Umsatz betrug 99 %.

### Beispiel 8

Pro Stunde wurden 11 g einer Lösung, die aus 20 Gew.-% 4-Formyltetrahydropyran und 80 Gew.-% Wasser bestand, in einen Verdampfer (200 bis 250°C) gepumpt und von dort mit einem Gemisch aus 17,5 l/h Stickstoff und 2,5 l/h Sauerstoff bei einer Reaktortemperatur von 150°C über 64 g Katalysator (Beispiel 3) in Rieselfahrweise geleitet. Der gasförmige Reaktionsaustrag wurde in Kühlfallen kondensiert und gaschromatographisch analysiert (quantitative Gaschromatographie). Es wurden 44 mol-% Tetrahydropyran-4-on gebildet. Der Aldehyd-Umsatz betrug 99 %.

### Beispiel 9

Pro Stunde wurden 11 g einer Lösung, die aus 20 Gew.-% 4-Formyltetrahydropyran und 80 Gew.-% Wasser bestand, in einen Verdampfer (200 bis 250°C) gepumpt und von dort mit einem Gemisch aus 17,5 l/h Stickstoff und 2,5 l/h Sauerstoff bei einer Reaktortemperatur von 150°C über 69 g Katalysator (Beispiel 4) in Rieselfahrweise geleitet. Der gasförmige Reaktionsaustrag wurde in Kühlfallen kondensiert und gaschromatographisch analysiert (quantitative Gaschromatographie). Es wurden 48 mol-% Tetrahydropyran-4-on gebildet. Der Aldehyd-Umsatz betrug 97 %.

## Patentansprüche

1. Verfahren zur Herstellung von cyclischen Etherketonen der allgemeinen Formel I 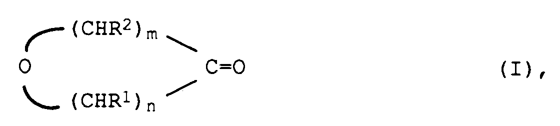 in der R¹ und R² Wasserstoff oder C₁- bis C₄-Alkyl, m und n ganze Zahlen von 1 bis 5 bedeuten, dadurch gekennzeichnet, daß man cyclische Etheraldehyde der allgemeinen Formel II 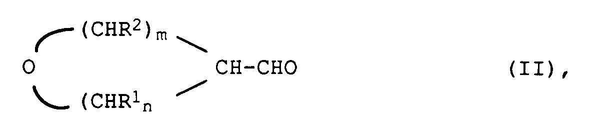 in der R¹, R², m und n die obengenannten Bedeutungen haben, mit sauerstoffenthaltenden Gasen in Gegenwart eines Voll- oder Trägerkatalysators, der Kupfer und/oder Mangan enthält, bei Temperaturen von 50 bis 300°C und Drücken von 0,01 bis 10 bar umsetzt.

2. Verfahren zur Herstellung von cyclischen Etherketonen I nach Anspruch 1, dadurch gekennzeichnet, daß man Trägerkatalysatoren einsetzt, bei der die Summe des Kupfer- und/oder Mangan-Anteils 0,1 bis 50 Gew.-% beträgt.

3. Verfahren zur Herstellung von cyclischen Etherketonen I nach Anspruch 1, dadurch gekennzeichnet, daß man Trägerkatalysatoren einsetzt, bei denen die Summe des Kupfer- und/oder Mangan-Anteils 0,1 bis 50 Gew.-% beträgt und die 0,4 bis 40 Gew.-% Zinkoxid enthalten.

4. Verfahren zur Herstellung von cyclischen Etherketonen I nach Anspruch 1, dadurch gekennzeichnet, daß man Trägerkatalysatoren aus Trägermaterialien von Aluminiumoxid, Siliziumdioxid, Titandioxid, Zirkoniumdioxid, Aluminiumsilikate, Magnesiumsilikate oder deren Gemische einsetzt.

5. Verfahren zur Herstellung von cyclischen Etherketonen I nach Anspruch 1, dadurch gekennzeichnet, daß man bei Temperaturen von 100 bis 250°C arbeitet.

6. Verfahren zur Herstellung von cyclischen Etherketonen I nach Anspruch 1, dadurch gekennzeichnet, daß das molare Verhältnis von Sauerstoff in den Sauerstoff enthaltenden Gasen zum Aldehyd 1 : 1 bis 200 : 1 beträgt.

7. Verfahren zur Herstellung von cyclischen Etherketonen I nach Anspruch 1, dadurch gekennzeichnet, daß man als Sauerstoff enthaltendes Gas Luft einsetzt.

8. Verfahren zur Herstellung von cyclischen Etherketonen I nach Anspruch 1, dadurch gekennzeichnet, daß man als inertes Verdünnungsmittel Stickstoff, Wasserdampf oder deren Gemische zusetzt.

9. Verfahren zur Herstellung von cyclischen Etherketonen I nach Anspruch 1, dadurch gekennzeichnet, daß R¹ und R² Wasserstoff, m und n jeweils die Zahl 2 in den Formeln I und II bedeuten.

## Claims

1. A process for preparing cyclic ether ketones of the formula (I) 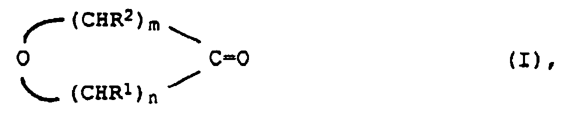 where R¹ and R² are hydrogen or C₁-C₄-alkyl, m and n are integers from 1 to 5, which comprises reacting cyclic ether aldehydes of the formula II 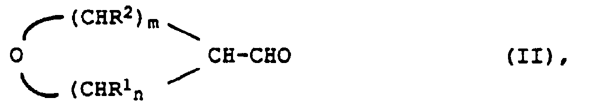 where R¹, R², m and n are as defined above, with oxygen-containing gases at from 50 to 300°C and pressures of from 0.01 to 10 bar in the presence of an unsupported or supported catalyst comprising copper and/or manganese.

2. A process for preparing cyclic ether ketones I as claimed in claim 1, wherein supported catalysts in which the sum of the copper and/or manganese content of from 0.1 to 50% by weight are used.

3. A process for preparing cyclic ether ketones I as claimed in claim 1, wherein supported catalysts in which the sum of the copper and/or manganese content is from 0.1 to 50% by weight and which contain from 0.4 to 40% by weight of zinc oxide are used.

4. A process for preparing cyclic ether ketones I as claimed in claim 1, wherein supported catalysts based on support materials comprising aluminum oxide, silicon dioxide, titanium dioxide, zirconium dioxide, aluminum silicates, magnesium silicates or mixtures thereof are used.

5. A process for preparing cyclic ether ketones I as claimed in claim 1 carried out at from 100 to 250°C.

6. A process for preparing cyclic ether ketones I as claimed in claim 1, wherein the molar ratio of oxygen in the oxygen-containing gases to the aldehyde is from 1:1 to 200:1.

7. A process for preparing cyclic ether ketones I as claimed in claim 1, wherein air is used as oxygen-containing gas.

8. A process for preparing cyclic ether ketones I as claimed in claim 1, wherein nitrogen, water vapor or a mixture thereof is added as inert diluent.

9. A process for preparing cyclic ether ketones I as claimed in claim 1, wherein R¹ and R² are hydrogen and m and n are each 2 in the formulae I and II.

## Revendications

1. Procédé de préparation d'éther-cétones cycliques de formule générale I 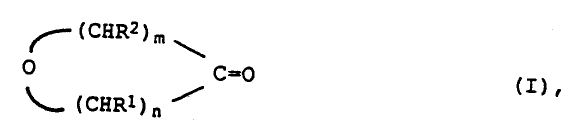 dans laquelle R¹ et R² représentent des atomes d'hydrogène ou des groupements alkyle en C₁-C₄, m et n représentent des nombres entiers de 1 à 5, caractérisé en ce que l'on fait réagir des éther-aldéhydes de formule générale II 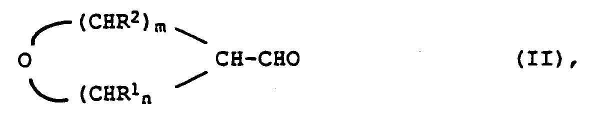 dans laquelle R¹, R², m et n ont les significations susmentionnées, avec des gaz contenant de l'oxygène en présence d'un catalyseur massif ou supporté contenant du cuivre et/ou du manganèse, à des températures de 50 à 300°C et sous des pression de 0,01 à 10 bar.

2. Procédé de préparation d'éther-cétones cycliques I selon la revendication 1, caractérisé en ce que l'on utilise un catalyseur supporté pour lequel la somme de la proportion du cuivre et/ou du manganèse s'élève à 0,1-50% en poids.

3. Procédé de préparation d'éther-cétones cycliques I selon la revendication 1, caractérisé en ce que l'on utilise un catalyseur supporté pour lequel la somme de la proportion du cuivre et/ou du manganèse s'élève à 0,1-50% en poids et contenant 0,4-40% en poids d'oxyde de zinc.

4. Procédé de préparation d'éther-cétones cycliques I selon la revendication 1, caractérisé en ce que l'on utilise un catalyseur supporté à base de matériaux supports en oxyde d'aluminium, dioxyde de silicium, dioxyde de titane, dioxyde de zirconium, silicate d'aluminium, silicate de magnésium ou de leurs mélanges.

5. Procédé de préparation d'éther-cétones cycliques I selon la revendication 1, caractérisé en ce que l'on travaille à des températures de 100 à 250°C.

6. Procédé de préparation d'éther-cétones cycliques I selon la revendication 1, caractérisé en ce que le rapport molaire de l'oxygène des gaz contenant de l'oxygène à l'aldéhyde s'élève de 1:1 à 200:1.

7. Procédé de préparation d'éther-cétones cycliques I selon la revendication 1, caractérisé en ce que l'on utilise de l'air en tant que gaz contenant de l'oxygène.

8. Procédé de préparation d'éther-cétones cycliques I selon la revendication 1, caractérisé en ce que l'on ajoute en tant que diluant inerte de l'azote, de la vapeur d'eau ou leurs mélanges.

9. Procédé de préparation d'éther-cétones cycliques I selon la revendication 1, caractérisé en ce que R¹ et R² représentent des atomes d'hydrogène et en ce que m et n représentent chacun le nombre 2 dans les formules I et II.
